# EUROPEAN PATENT SPECIFICATION

(11) **EP 1 919 469 B1**
(45) Date of publication and mention of the grant of the patent: **16.02.2011**
(21) Application number: 06792689.9
(22) Date of filing: 03.08.2006
(51) Int. Cl.: A61K 31/41, A61P 9/12

(54) **PROCESS FOR PREPARING AN ANGIOTENSIN II RECEPTOR ANTAGONIST**
VERFAHREN ZUR HERSTELLUNG EINES ANGIOTENSIN-II-REZEPTOR-ANTAGONISTEN
PROCEDE DE PREPARATION D'UN ANTAGONISTE DES RECEPTEURS DE L'ANGIOTENSINE II

(30) Priority: 04.08.2005 EP 05381040; 04.08.2005 US 705827 P
(43) Date of publication of application: 14.05.2008
(73) Proprietor: Farmaprojects, S.A., 08902 L'Hospitalet de Llobregat (Barcelona) (ES)
(72) Inventor: BESSA BELMUNT, Jordi, E-08902 L'hospitalet de Llobregat (ES)
(74) Representative: ZBM Patents
(86) International application number: PCT/EP2006/065056
(87) International publication number: WO 2007/017469

(56) References cited:
- EP-A- 0 454 511
- WO-A-93/08169
- WO-A-99/06398
- WO-A-2004/007482
- WO-A-2004/065383
- WO-A-2004/072064
- WO-A-2005/051943
- DE-A1- 4 313 747
- US-A- 4 675 403
- G. WINTERS ET AL: "Ricerche su 3-oxa-1-azaspiro-[4.4]-non-1-en-4-oni 2-sostituiti" IL FARMACO, EDIZIONE SCIENTIFICA, vol. 21, no. 9, 1966, pages 624-630, XP008059841 cited in the application

## Description

### FIELD OF THE INVENTION

The present invention relates to a process for preparing an angiotensin II receptor antagonist, in particular irbesartan, and protected forms for the preparation thereof. It also relates to new intermediates that are useful for the preparation of angiotensin II receptor antagonists.

### BACKGROUND ART

Irbesartan is an angiotensin II receptor antagonist of formula:

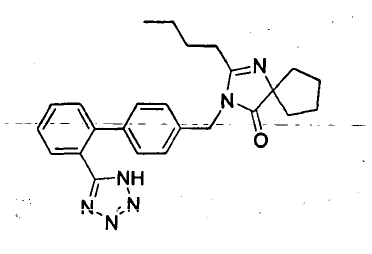

Angiotensin II is a peptide hormone that is a potent vasopressor. It is the biologically active product of the renin-angiotensin system. Renin acts on the angiotensinogen of the plasma to produce angiotensin I, which is converted to angiotensin II by the action of the angiotensin I converting enzyme.

Irbesartan inhibits the action of angiotensin II on its receptors and thus prevents the increase in blood pressure produced by the hormone-receptor interaction. It is therefore useful in the treatment of hypertension and heart failure.

Several synthetic routes have been described in the literature for the preparation of irbesartan. Most of the routes comprise the reaction of a bromomethylbiphenyl or an aminomethylbiphenyl compound with 2-butyl-1,3-diazaspiro[4,4]non-1-en-4-one (e.g. EP454511, WO2005051943, WO9906398, WO2004007482). The last step of the processes described in said first three patent applications corresponds to the formation of the tetrazole ring from a cyano group employing an azide derivative.

There have also been described processes for preparing irbesartan in which the last step corresponds to the formation of the biphenyl moiety. Thus, WO2004065383A2 describes a process for preparing irbesartan by a Suzuki coupling reaction comprising the reaction of a bromobenzyl spiro compound with a 2-(tetrazol-5-yl)phenylboronic acid derivative in the presence of a palladium catalyst and triphenyl phosphine in 1,2-dimethoxyethane (DME) and tetrahydrofuran.

WO2004072064A1 discloses different routes for the synthesis of irbesartan, in which the last step is the formation of the spiro cycle, followed by deprotection. The formation of the spirocycle is described:
- By reaction of 1-pentanamidocyclopentanecarboxamide with 5-(4'-bromomethylbiphenyl-2-yl)-trityl-1*H-*tetrazole,
- by reaction of an amino compound with an imidate intermediate, under inert atmosphere in dry toluene,
- by reaction of a valeramide derivative in the presence of oxalyl chloride and 2,6-lutidine with an amine under argon.

### SUMMARY OF THE INVENTION

The problem to be solved by the present invention is to provide an efficient alternative process for preparing irbesartan.

The solution is based on the fact that the present inventors have identified a simplified process for preparing irbesartan. Said process comprises the reaction between an oxazolone and a primary amine, in particular, between the spiro compound 2-butyl-3-oxa-1-azaspiro[4.4]non-1-en-4-one and an aminomethylbiphenyl intermediate. Said spiro compound has only been found to be described in an article of 1966 (c.f. Winters, G. et al., Farmaco, Edizione Scientifica (1966), 21 (9), 624-30). Surprisingly its use for the manufacture of irbesartan, according to the process of the invention, has been found to be advantageous. See working examples 1-7 herein for a further description.

Accordingly, a first aspect of the invention relates to a process for preparing a compound of formula (I) or a pharmaceutically acceptable salt thereof 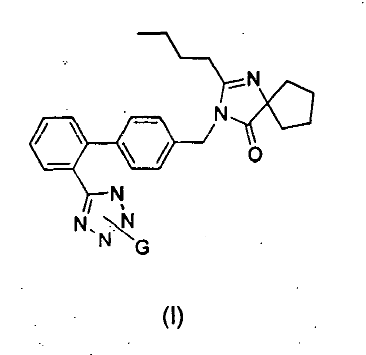 wherein:
G is H or a tetrazole protecting group,
comprising the reaction between an intermediate of formula (II) or an acid addition salt thereof 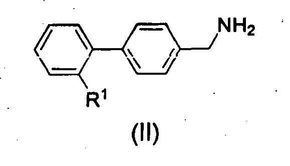 wherein:
R¹ is a tetrazolyl group or an intermediate or protected form that can be transformed into a tetrazolyl group
and an intermediate of formula (III) 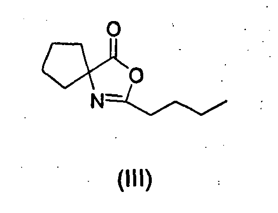 in an appropriate solvent system and thereafter as necessary transforming said intermediate or protected forms of R¹ into a tetrazolyl group and, if desired, converting said compound of formula (I) into a pharmaceutically acceptable salt thereof.

The process of the present invention presents several advantages that are important for the manufacture of irbesartan on an industrial scale. It is a simplified process that renders irbesartan in one step from intermediates easy to obtain from commercial products. The reaction is selective for the primary amine, and presents no interaction with the NH group of the tetrazole ring. Thus, advantageously, the reaction proceeds with high yields even if no protecting groups (e.g. the trityl group for protecting the tetrazole ring) are employed.

Advantageously, when the process is carried out without protecting groups, additional steps of protection and deprotection are avoided and also the need of inflating the mass, which has to be subsequently deflated. This atomic economy is an important advantage for a manufacturing method on an industrial scale.

A further advantage of the process is that irbesartan may be obtained from commercial products without the need of handling explosive and highly toxic reagents, such as azide derivatives.

### Definitions

Acid addition salts of compounds of formula (II) or of formula (V) refer to amino salts formed with inorganic and organic acids such hydrochlorides, hydrobromides, sulphates, nitrates, phosphates, organic sulfonates, among others.

By an intermediate form that can be transformed into a tetrazolyl group it is meant herein a group such as a cyano group, that can be transformed to a tetrazolyl group by methods well known to those skilled in the art.

By a protected form that can be transformed into a tetrazolyl group it is meant in the present invention, a tetrazole ring protected with a tetrazole protecting group.

By a leaving group X, it is meant in the present invention, a detachable group in the reaction conditions (e.g. X is a good leaving group in the conditions of a Suzuki coupling, L is a good leaving group that can be displaced with a tertiary amine). Thus, the leaving group comprises an atom of CI, Br, l, a methanesulfonyloxy, toluensulfonyloxy, benzenesulfonyloxy or trifluoromethanesulfonyloxy group. Preferably X is an atom of Cl, Br, l, or a trifluoromethanesulfonyloxy group.

By a C₁-C₆ linear or branched alkyl it is meant in the present invention a linear or branched alkyl group which contains up to 6 carbon atoms. Thus it comprises, for instance, methyl, ethyl, propyl, isopropyl, butyl, isobutyl, sec-butyl, *tert*-butyl*,* n-pentyl, 1,2-dimethyl propyl, 1,1-dimethyl propyl, 2,2-dimethyl propyl, 2-ethyl propyl, n-hexyl, 1,2-dimethyl butyl, 2,3-dimethyl butyl, 1,3-dimethylbutyl, 1-ethyl-2-methylpropyl, and 1-methyl-2-ethyl propyl groups.

By a (C₁-C₄)-alkoxy it is meant in the present invention a linear or branched alkoxy group which contains up to 4 carbon atoms. Thus it comprises, for instance, methoxy, ethoxy, propoxy, isopropoxy, butoxy, isobutoxy, sec-butoxy and *tert*-butoxy groups.

By a (2'-(1*H*-tetrazol-5-yl) biphenyl-4-yl) methanamine moiety it is meant herein a (2'-(1*H*-tetrazol-5-yl) biphenyl-4-yl) methanamine wherein the N atom of said amine moiety may form part of an heterocyclic group.

### DETAILED DESCRIPTION OF PARTICULAR EMBODIMENTS

As described above, in the process according to the first aspect of the invention, intermediate of formula (III) may react with an intermediate of formula (II) or an acid addition salt thereof. Preferably, intermediate of formula (III) is reacted with intermediate of formula (II), or its hydrochloride.

The best conditions to carry out the process vary according to the parameters considered by the person skilled in the art, Such as the solvents, temperature and similar. Such reaction conditions may be easily determined by the person skilled in the art by routine tests, and with the teaching of the examples included in this document.

The reaction may be carried out in different solvents systems. Preferably, the solvent system is an organic solvent or a mixture of organic solvents. The organic solvent may be selected from aliphatic or aromatic (C₆-C₈) hydrocarbons such as toluene, xylene; aliphatic ethers such as dimethoxyethane, diethoxymethane, diglyme, dioxane, and tetrahydrofuran, and aliphatic (C₁-C₅) alcohols such as methyl, ethyl, propyl, isopropyl, butyl, isobutyl, tert-butyl, pentyl, isopentyl and tert-pentyl alcohol; ketones such as acetone, methylethylketone, or a polar aprotic solvent. Preferably, the solvent system comprises a polar aprotic solvent, since, especially when R¹ is H, the reaction proceeds faster when it is carried out in presence of a polar aprotic solvent. Polar aprotic solvents that may be suitable for the reaction include: N- dialkylated amides such as *N*,*N*-dimethylformamide (DMF), 1-methylpyrrolidone (NMP), *N,N*-dimethylacetamide (DMA), dioxane and dimethyl sulfoxide (DMSO). In a preferred embodiment, the reaction is carried out in presence of DMF or NMP.

The reaction between the intermediate of formula (II) or an acid addition salt thereof and intermediate of formula (III) is preferably carried out in a neutral medium or in the presence of an acid catalyst. By neutral medium it is meant herein a medium without the presence of any acidic or basic agent. In a preferred embodiment, it is carried out in the presence of an acid catalyst, since, advantageously, it generally leads to higher yields. Suitable acid catalysts include: inorganic acids such as hydrochloric acid, hydrobromic acid, phosphoric acid, sulfuric acid, nitric acid and boric acid; organic sulfonic acids such as methanesulfonic acid, p-toluenesulfonic acid, camphorsulfonic acid, pyridinium p-toluenesulfonate, etc; and Lewis acids such as aluminum trichloride, boron trifluoride, zinc dichloride, tin tetrachloride, etc. In a more preferred embodiment, the acid catalyst is selected from the group consisting of methanosulfonic acid, *p*-toluensulfonic acid and hydrochloric acid.

In a preferred embodiment, the reaction is carried out at a temperature comprised between 100 °C and 180 °C.

When R¹ of the intermediate of formula (II) is an intermediate form that can be transformed into a tetrazolyl group, the process further comprises the conversion of said intermediate form into a tetrazolyl group. In one embodiment, said intermediate form is a cyano group. But other intermediate forms that can be transformed into a tetrazolyl group may be used, such as an hydrazinoiminomethyl group. These intermediate forms may be converted into the tetrazole by methods known by those skilled in the art. For instance, when it is a cyano group, it can be transformed by several procedures using hydrazoic acid (e.g. by heating sodium azide and ammonium chloride as described in J. P. Hurwitz y A. J. Tomson, J. Org. Chem., (1961), 26, 3392). Preferably the tetrazole is prepared by the 1,3-bipolar cycloaddition of trialkyltin or triaryltin azides to the nitrile, as described in e.g. EP475898 or WO9906398.

When R¹ of the intermediate of formula (II) is a protected form that can be transformed into a tetrazolyl group, and G is H, the process comprises a further step in which the protective group is cleaved from the tetrazole ring. The protective group of the tetrazole ring can be removed by procedures known in the art (cf. Protective Groups in Organic Synthesis, Wiley-Interscience, (1999)). For instance, when trityl group is used as the protective group of the tetrazole ring, it can be deprotected either in acidic or basic conditions. Preferably, the deprotection is carried out in acidic conditions, for example, HCl in a suitable solvent such as methanol or a mixture of dioxane/water.

Preferably, the process according to the first aspect of the invention is carried out without the use of protecting groups. Thus, in a preferred embodiment G is H and R¹ is tetrazole.

The compound of formula (I) obtained by the process according to the first aspect of the invention, may be converted to a pharmaceutically acceptable salt thereof by methods well known to those skilled in the art.

The intermediate of formula (III) may be prepared by methods described in the literature (c.f. Winters, G. et al., Farmaco, Edizione Scientifica (1966), 21 (9), 624-30). The method described therein comprises a two step process starting from cycloleucine that renders the product with a poor yield. The present inventors have found a new and simplified method that renders the intermediate of formula (III) in one step, with high yields. Thus, an embodiment of the invention relates to a process for preparing an intermediate of formula (III) wherein it is prepared by reaction between cycloleucine and valeroyl chloride. The reaction is carried out in an appropriate solvent system, preferably in aprotic solvents, such as toluene or tetrahydrofuran (THF), and in presence of a base able to capture the hydrochloric acid released during the reaction, such as trialkylamines, Hünig's bases or inorganic bases such as carbonates or hydroxides, preferably triethylamine or diisopropylethylamine. It may be carried out at a temperature comprised between 20 and 150°C, preferably between 50 and 110°C.

In a preferred embodiment of the first aspect of the invention, the intermediate of formula (III) is prepared according to the above mentioned embodiment of the invention.

The intermediate of formula (II) may be prepared by several methods described in the literature, such as those described in WO9906398, WO9308169, WO9316049, EP540356 and EP542554.

The present inventors have also identified a new process for the preparation of intermediate of formula (II) by a Suzuki coupling reaction. Thus, as described above, an intermediate of formula (II) may be prepared by reaction between an intermediate of formula (IV) 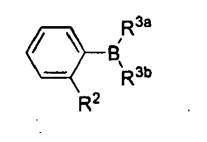 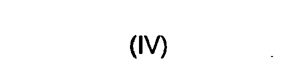 wherein:
R² is a tetrazolyl group or an intermediate or protected form that can be transformed into a tetrazolyl group,
R^{3a} and R^{3b} are each independently selected from the group consisting of: Cl, Br, (C₁-C₄)-alKoxy, hydroxy, or alternatively,
R^{3a} and R^{3b} can be taken together with the B atom to form a cyclic structure selected from one of the followings 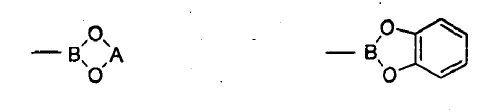
wherein A is (CH₂)ₙ and n is an integer from 2 to 4,
and an intermediate of formula (V) or an acid addition salt thereof 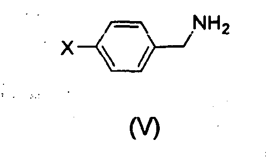 wherein:
X is a leaving group,
in the presence of a base, a metallic catalyst and an appropriate solvent system and optionally transforming said intermediate or protected form of R² into a tetrazolyl group and if desired converting the compound of formula (II) into an acid addition salt thereof.

In a preferred embodiment of the first aspect of the invention, the intermediate of formula (II) is prepared according to the above mentioned embodiment of the invention.

Different solvent systems may be appropriate for the reaction. Preferably, the solvent system is selected from water, an organic solvent and mixtures of water and one or more organic solvents. Preferably, the solvent is selected from C₁-C₄ alcohols, DMF, DME, THF, and their mixtures with water. In a more preferred embodiment, the solvent system is selected from the group consisting of DMF and DME.

A variety of bases may be used in the process. Suitable bases may be seiecied from organic and inorganic bases. Preferably, the base is selected from alkaline hydroxides and alkaline carbonates. More preferably, the base is selected from sodium hydroxide and potassium hydroxide.

The suitable metallic catalysts include catalysts of palladium (0) or nickel, such as, tetrakis(triphenylphosphine)palladium (0), Bis(triphenylphosphine)palladium (II) dichloride, a complex formed by palladium acetate or palladium chloride or Pd/C with triaryl or trialkylphosphines optionally substituted, (1,3-bis[diphenylphosphino]propane)dichloronickel (II) (Ni(dppp)Cl₂), dichloro[1,1'-bis(diphenylphosphino)ferrocene]nickel (II) (Ni(dppf)Cl₂). Preferably, the metallic catalyst is selected from tetrakis(triphenylphosphine)palladium (0),a complex formed by palladium chloride with triphenylphosphine.

Preferably, the leaving group X is selected from an atom of halogen (Cl, Br,l), and a trifluoromethanesulfonyloxy group.

In a preferred embodiment, R³and R^{3b} are hydroxy and R² is a tetrazolyl group.

Suitable protecting groups for the tetrazole ring, procedures for introducing them and removing them are described in Greene and Wuts (Protective Groups in Organic Synthesis, Wiley and Sons, 1999).

In a preferred embodiment R¹ is a tetrazolyl group.

Intermediate of formula (IV), when R¹ is a tetrazolyl group may be obtained as described in the literature (e.g. according to example II of DE4313747 patent).

In a still more preferred embodiment, the intermediate of formula (IIa), 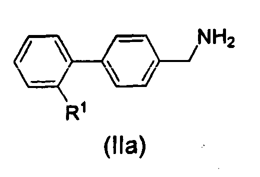 wherein R¹ is a tetrazolyl group or a protected form that can be transformed into a tetrazolyl group, is prepared in a "one-pot" process from an intermediate of formula (VI) 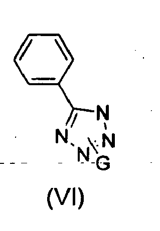 wherein G is as defined above. Thus, the process for preparing the intermediate of formula (IIa), according to the embodiment described above, in a still more preferred embodiment, further comprises previously preparing "in situ" an intermediate of formula (IV), by reaction of an intermediate of formula (VI), with an alkyllithium compound of formula R⁴-Li wherein R⁴ is a C₁-C₆ linear or branched alkyl and a boronic ester of formula B(OR⁵)₃ wherein R⁵ is a (C₁-C₄)-alkyl group.

This reaction may be carried out in anhydrous aprotic solvents, such as THF, diethylether or 1,2-dimethoxyethane.

Preferably, the alkyllithium compound R²-Li is selected from hexyllithium and butyllithium. Preferably, R⁵ in the boronic ester is a methyl or isopropyl group. Preferably, G in the compound of formula (VI) is H.

The present inventors have also identified another new process for the preparation of intermediate of formula (II) by an efficient conversion of a leaving group into a primary amine. Thus, as described above, an intermediate of formula (II) may be prepared by reaction of an intermediate of formula (VII) 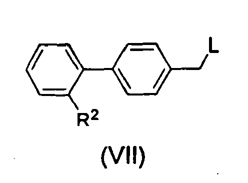 wherein:
R² is a tetrazolyl group or an intermediate or protected form that can be transformed into a tetrazolyl group,
L is a leaving group,
with hexamethylenetetramine in presence of an appropriate solvent system to afford the compound of formula (VIII): 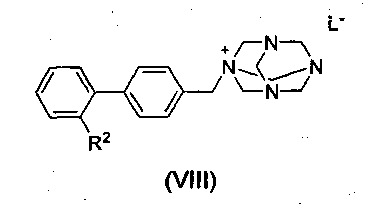 wherein
R² is as defined above and
L⁻ is the corresponding anion of the leaving group L,
and transform this compound in acid media to afford compound (II) and optionally transforming said intermediate or protected form of R² into a tetrazolyl group and if desired converting the compound of formula (II) into an acid addition salt thereof.

In a preferred embodiment of the first aspect of the invention, the intermediate of formula (II) is prepared according to the above mentioned embodiment of the invention.

Different solvent systems may be appropriate for the reaction. Preferably, the solvent system is an aprotic solvent selected from ketones such as acetone, methylethylketone; aliphatic ethers such as dimethoxyethane, diethoxymethane, diglyme, dioxane, and tetrahydrofuran; aliphatic or aromatic (C₆-C₈) hydrocarbons such as toluene, xylene; esthers,such as ethyl or butyl acetate. More preferably, the solvent system comprises a polar aprotic solvent, most preferably, a ketone.

Preferably the reaction is carried out at a temperature comprised between 25 and 100°C More preferably, from 40-70°C.

The leaving group is preferably selected from the group consisiting of Cl⁻ , Br⁻ , l⁻, a methanesulfonate, p-toluensulfonate, benzenesulfonate optionally substituted by nitro groups and trifluoromethanesulfonate. More preferably it is selected from Cl, Br, l. Yet more preferably the leaving group is Br.

The compound of formula (VIII) may be transformed into the compound of formula (II) in acid media. The acid media may be achieved by addition of an inorganic acid, such as a hydrogen halide (e.g. hydrogen chloride, hydrogen bromide, hydrogen iodide). More preferably, it is hydrogen chloride. This transformation is carried out in a suitable solvent system. Preferably the solvent system comprises an aliphatic (C₁-C₅) alcohol such as methyl, ethyl, propyl, isopropyl, butyl, isobutyl, tert-butyl, pentyl, isopentyl and tert-pentyl alcohol, and their mixtures with water. More preferably it is carried out in an aqueous alcohol media.

In a preferred embodiment, a compound of formula (II), wherein R¹ is a tetrazolyl group, is obtained from a compound of formula (VIII), wherein R² is a trityl protected tetrazole and in only one hydrolysis/deprotection step with hydrogen chloride in aqueous alcohol media.

If desired, intermediate of formula (II) may be converted into an acid addition salt thereof. The addition salts, where applicable, can be prepared by treatment with acids, such as hydrochloric, hydrobromic, sulphuric, nitric, phosphoric, alkyl or arylsulfonic, in water or organic solvents such as ethers, alcohols, ketones, esters, or mixtures of solvents.

Compound VIII is useful for preparing intermediates of formula (II), which are in turn useful for preparing compounds of formula (I), as illustrated by the process disclosed in the present invention, as well as for preparing an angiotensin II receptor antagonist with a (2'-(1*H-*tetrazol-5-yl) biphenyl-4-yl) methanamine moiety. Thus, compound (VIII), may be used for preparing an angiotensin II receptor antagonist with a (2'-(1*H*-tetrazol-5-yl) biphenyl-4-yl) methanamine moiety. Preferably said angiotensin II receptor antagonist with a (2'-(1*H*-tetrazol-5-yl) biphenyl-4-yl) methanamine moiety is selected from the group consisting of irbesartan and tasosartan.

Intermediate of formula (II) may be transformed into an angiotensin II receptor antagonist with a (2'-(1*H*-tetrazol-5-yl) biphenyl-4-yl) methanamine moiety, by processes known in the art (e.g. as described in EP454511 for irbesartan, and in EP539086 for tasosartan). In the process according to the invention,, intermediate of formula (II) is preferably transformed into irbesartan or tasosartan. More preferably it is transformed into ibesartan.

In the compounds of formula (VIII), L⁻ is preferably selected from the group consisting of Cl⁻, Br' , l⁻, a methanesulfonate, p-toluensulfonate, benzenesulfonate and trifluoromethanesulfonate. Preferred compounds of formula (VIII) are those wherein L⁻ is Br⁻ and R² is a tetrazolyl group or a tetrazolyl group protected with a trityl group.

The intermediates of formula (VIII) may be prepared, as described above, by reaction of an intermediate of formula (VII) with hexamethylenetetramine in presence of an appropriate solvent system. Compounds of formula (VII) are commercially available or may be readily prepared from available commercial products by methods well known in the art.

Throughout the description and claims the word "comprise" and variations of the word, such as "comprising", are not intended to exclude other technical features, additives, components, or steps. The following examples are provided by way of illustration.

### EXAMPLES

### EXAMPLE 1

### (2-(1H-tetrazol-5-yl) biphenyl-4-yl)methanamine hydrochloride

0.42 g (2.21 mmol) of 2-(1*H-*tetrazol-5-yl)phenylboronic acid (obtained according to example II of DE4313747 patent), 0.49 g (2.20 mmol) of (4-bromophenyl)methanamine hydrochloride, 0.53 g (13.25 mmol) of sodium hydroxide and 0.13 g (0.112 mmol) of tetrakis(triphenylphosphine)palladium (0) were added to a mixture of 5 mL of DMF and 0.5 mL of water. Nitrogen was bubbled to the resultant mixture for 1 min and the reaction was heated to 100°C under nitrogen atmosphere for 7 h. Once the DMF had been removed by distillation under reduced pressure, 5 mL of water were added and the mixture was washed twice using 5 mL of ethyl acetate each time. The aqueous phase was adjusted to pH si with concentrated hydrochloric acid and washed again three times with 5 mL of ethyl acetate. Remains of ethyl acetate were distilled from the aqueous phase and the mixture was left to precipitate for 1 h at room temperature and later for 1 h at 0 °C. The solid was filtered, washed with cold water and dried under reduced pressure at 60°C affording (2'-(1*H*-tetrazol-5-yl) biphenyl-4-yl) methanamine hydrochloride.
RMN ¹H (DMSO), δ(ppm): 4.00 (d, 2H, Ar-CH₂-); 7.14 (d, 2H, ArH); 7.40 (d, 2H, ArH); 7.52 (d, 1H, ArH); 7.59 (d, 1H, ArH); 7.65-7.72 (m, 2H, ArH); 8.37 (sa, 3H, -NH₃⁺C⁻).
M.P. = 283 °C (decomposes)

### EXAMPLE 2

### (2'-(1H-tetrazol-5-yl) biphenyl-4-yl) methanamine hydrochloride

Under nitrogen atmosphere, 6.6 mL of 1,2-dimethoxyethane were added to 0.66 g (4.52 mmol) of 5-phenyl-1*H*-tetrazole. The suspension was cooled to 0 °C and then 4.7 mL (10.81 mmol) of 2.3 M solution of hexyllithium in hexane was slowly added maintaining the temperature at between 0 and 10°C. After 1 h at 0 °C, 1.1 mL (9.81 mmol) of trimethyl borate was added and the reaction was left stirring at room temperature for a further 2 h. One mL of water, 1.08 g (27 mmol) of sodium hydroxide, 1 g (4.49 mmol) of (4-bromophenyl)methanamine hydrochloride and 0.208 g (0.18 mmol) of tetrakis(triphenylphosphine)palladium (0) were successively added and the mixture was heated to reflux under nitrogen atmosphere for 5 h. Afterwards, 5 mL of water were added and the resultant aqueous phase was washed three times with 5 mL of ethyl acetate. The aqueous phase was adjusted to pH 1 with concentrated hydrochloric acid and washed again three times with 5 mL of ethyl acetate. Remains of ethyl acetate were distilled from the aqueous phase and the mixture was left to precipitate for 1 h at room temperature and later for 1 h at 0 °C. The solid was filtered, washed with cold water and dried under reduced pressure at 60°C affording 0.64 g of a crude that can be crystallized from 2.5 mL of water to obtain pure (2'-(1*H*-tetrazol-5-yl) biphenyl-4-yl) methanamine hydrochloride.

### EXAMPLE 3

### N-(2'-(1-trityl-1H-tetrazol-5-yl)biphenyl-4-yl)methyl hexamethylenetetraminium bromide

To a suspension of 10 g (17.94 mmol) of 5-(4'-(bromomethyl)biphenyl-2-yl)-1-trityl-1*H*-tetrazole in 100 mL of acetone was added at 20-25°C 2.66 g (18.97 mmol) of hexamethylenetetramine. The mixture was heated to reflux for 2 h under nitrogen atmosphere. After cooling to 20-25°C, the mixture was filtered and the solid obtained was washed twice with 20 mL of acetone and dried under reduced pressure at 45°C affording 12.25 g (97.9%) of (2'-(1-trityl-1*H-*tetrazol-5-yl)biphenyl-4-yl)methanahexamethylenetetraminium bromide.
RMN ¹H (DMSO), δ(ppm): 4.01 (d, 2H, Ar-CH₂-); 4.34 (d, 3H, N-CH₂-N); 4.56 (d, 3H, N-CH₂-N); 5.02(s, 6H, N⁺-CH₂-N); 6.84(d, 6H, trityl); 7.21 (d, 2H, ArH); 7.27-7.43(m, 11H, trityl + ArH); 7.48 (d, 1H, ArH); 7.52-7.72 (m, 2H, ArH); 7.86 (d,1H, ArH).
RMN ¹³C (DMSO), δ(ppm): 48.79, 59.00, 70.05, 77.99, 82.51,124.41, 125.81, 128.11, 128.57, 129.71, 130.54, 130.81, 132.31, 140.76, 141.00, 142.14, 163.50.
M.P. = 142 °C (decomposes)

### EXAMPLE 4

### (2'-(1H-tetrazol-5-yl) biphenyl-4-yl) methanamine hydrochloride

To a heated suspension of 12.25 g (17.55 mmol) of *N*-(2'-(1-trityl-1*H*-tetrazol-5-yl)biphenyl-4-yl)methyl hexamethylenetatraminium bromide in 110 mL of ethanol under reflux, was added 10.2 mL (122.85 mmol) of HCl 37%. The mixture was left under reflux for 1 h and then cooled directly to 0°C, filtrated and washed twice with 10 mL of ethanol. The filtrate was distilled under reduced pressure and the residue was treated with 60 mL of acetone. The precipitate was filtered and washed twice with 10 mL of acetone and dried under reduced pressure at 45°C affording 5.11 g of crude (2'-(1*H*-tetrazol-5-yl) biphenyl-4-yl) methanamine hydrochloride. 2.5 g of the crude was crystallized from 25 mL of ethanol to obtain 1.87 g (74.8%) of pure (2 '-(1*H-*tetrazol-5-yl) biphenyl-4-yl) methanamine hydrochloride.

### EXAMPLE 5

### 2-n-butyl-3-oxa-1-azaspiro-[4.4]-non-1-en-4-one

9.2 mL (77.5 mmol) of valeroyl chloride were added to a suspension of 5 g (38.7 mmol) of 1-aminocyclopentanecarboxylic acid in 50 mL of toluene. The mixture was heated to 80°C and 11.9 mL (85.4 mmol) of triethylamine were slowly added over one hour. The reaction was left for 4 h at 80°C and, after cooling to room temperature, was washed twice with 50 mL of water, twice with 25 mL of 10 % aqueous potassium carbonate solution, once with 25 mL saturated ammonium chloride solution and finally with 25 mL of water. The resultant organic phase was evaporated at a pressure of 200 mbar to obtain 10.16 g of yellowish oil that was purified by distillation at 10 mbar (130-140 °C) affording 5.62 g (74.4 %) of 2-n-butyl-3-oxa-1-azaspiro-[4.4]-non-1-en-4-one.
RMN¹H (CDCl₃), δ(ppm): 0.94 (t, 3H, -CH₂-CH₂-CH₂-CH₃); 1.32-1.47 (m, 2H, - CH₂-CH₂-CH₂-CH₃); 1.61-1.73 (m, 2H, -CH₂-CH₂-CH₂-CH₃); 1.82-2.08 (m, 8H, cyclopentane); 2.46 (t, 2 H, -CH₂-CH₂-CH₂-CH₃).

### EXAMPLE 6

### 2-n-butyl-3-[[2'-(tetrazol-5-yl)biphenyl4-yl]-methyl]-1,3 diazaspiro[4.4]non-1-en-4-one

Under nitrogen atmosphere, a solution of 1.03 g (5.27 mmol) of 2-n-butyl-3-oxa-1-azaspiro-[4.4]-non-1-en-4-one in 1 mL of N-methylpirrolidone was added over 30 min to a mixture of 1.0 g (3.47 mmol) of (2'-(1*H*-tetrazol-5-yl) biphenyl-4-yl) methanamine hydrochloride in 3 mL of N-methylpirrolidone heated at 140°C. The reaction was left for 5 h at 140°C and 200 mbar and after cooling to room temperature 15 mL of 10 % aqueous sodium hydroxide solution was added. Then, 15 mL of ethyl acetate were added and the mixture was acidified with concentrated hydrochloric acid until pH 4-5. After a while, the mixture started to precipitate and after leaving for 1 h at 20-25 °C the solid was filtered, washed with water and ethyl acetate and, finally, the solid was dried under reduced pressure at 65°C affording 0.90 g (60.4 %) of 2 - n-butyl -3-[[2'-(tetrazol-5-yl)biphenyl-4-yl]-methyl]-1,3-diazaspiro[4.4] non-1-en-4-one.
RMN ¹H (CDCl₃), δ(ppm): 0.82 (t, 3H, -CH₂-CH₂-CH₂-CH₃); 1.18-1.33 (m, 2H, - CH₂-CH₂-CH₂-CH₃); 1.40-1.52 (m, 2H, -CH₂-CH₂-CH₂-CH₃); 1.62-1.86 (m, 8H, cyclopentane); 2.17 (t, 2 H, -CH₂-CH₂-CH₂-CH₃); 4.65 (s, 2H, Ar-CH₂-); 7.04 (d, 2H, ArH); 7.15 (d, 2H, ArH); 7.44 (dd, 1H, ArH); 7.49-7.65 (m, 2H, ArH); 7.87 (dd, 1H, ArH).
M.P.=182°C

### EXAMPLE 7

### 2-n-butyl-3-[[2'-(tetrazol-5-yl)biphenyl-4-yl]-methyl]-1,3-diazaspiro[4.4]non-1-en-4-one

A solution of 0.44 g (2.25 mmol) of 2-n-butyl-3-oxa-1-azaspiro-[4.4]-non-1-en-4-one in 0.4 mL of N-methylpirrolidone was added over 10 min to a mixture of 0.4 g (1.39 mmol) of (2'-(1H-tetrazol-5-yl)biphenyl-4-yl)methanamine hydrochloride and 0.08 g (0.83 mmol) of methanesulfonic acid in 1.6 mL of N-methylpirrolidone heated at 160 °C. The reaction was left for 2.5 h at 160°C and 200 mbar and after removing the N-methylpirrolidone by distillation under reduced pressure and cooling to room temperature 4 mL of 10 % aqueous sodium hydroxide solution was added. Then, 6 mL of ethyl acetate were added and the mixture was acidified with concentrated hydrochloric acid until pH 4-5. After a while, the mixture started to precipitate and after leaving for 1 h at 20-25 °C the solid was filtered, washed with water and ethyl acetate and, finally, the solid was dried under reduced pressure at 60°C affording 0.42 g (70,5 %) of 2 - n-butyl - 3 - [[2 ' - (tetrazol - 5 - yl) biphenyl - 4 - yl] - methyl] - 1,3 - diazaspiro [4.4] non - 1 - en - 4 - one.

## Claims

1. A process for preparing a compound of formula (I) or a pharmaceutically acceptable salt thereof 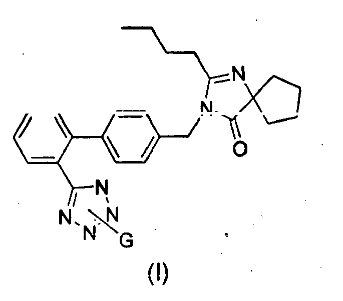 wherein:
G is H or a tetrazole protecting group,
comprising the reaction between an intermediate of formula (II) or an acid addition salt thereof 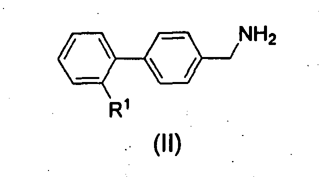 wherein:
R¹ is a tetrazolyl group or an intermediate or protected form that can be transformed into a tetrazolyl group
and an intermediate of formula (III) 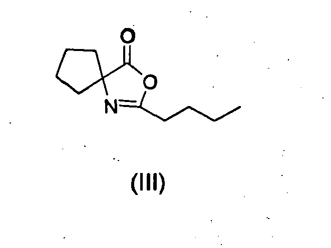 in an appropriate solvent system and thereafter as necessary transforming said intermediate or protected forms of R¹ into a tetrazolyl group and, if desired, converting said compound of formula (I) into a pharmaceutically acceptable salt thereof.

2. The process according to claim 1, wherein R¹ is a tetrazolyl group and G is H.

3. The process according to any of claims 1 or 2, wherein the reaction is carried out in the presence of an acid catalyst.

4. The process according to claim 3, wherein said acid catalyst is selected from the group consisting of: methanosulfonic acid, *p*-toluensulfonic acid, and hydrochloric acid.

5. The process according fo any of claims 1 to 4, Wherein said solvent system comprises a polar aprotic solvent.

6. The process according to any of the preceding claims, wherein the intermediate of formula (III) is prepared by reaction between cycloleucine and valeroyl chloride.

7. The process according to any of the preceding claims, wherein the intermediate of formula (II) or an acid addition salt thereof is prepared by reaction between an intermediate of formula (IV) 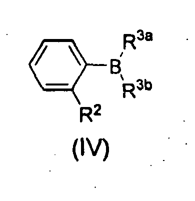 wherein:
R² is a tetrazolyl group or an intermediate or protected form that can be transformed to a tetrazolyl group,
R^{3a} and R^{3b} are each independently selected from the group consisting of: Cl, Br, (C₁-C₄)-alkoxy, hydroxy, or alternatively,
R^{3a} and R^{3b} can be taken together with the B atom to form a cyclic structure selected from the one of the followings 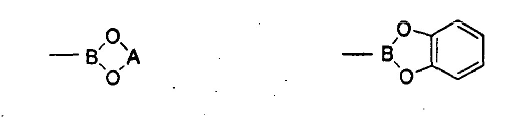
wherein A is (CH₂)ₙ and n is an integer from 2 to 4,
and an intermediate of formula (V) or an acid addition salt thereof 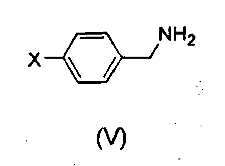 wherein:
X is a leaving group
in the presence of a base, a metallic catalyst and an appropriate solvent system and optionally transforming said intermediate or protected form of R² to a tetrazolyl group and if desired converting the compound of formula (II) to an acid addition salt thereof.

8. The process according to claim 7, wherein R^{3a} and R^{3b} are hydroxy and R² is a tetrazolyl group.

9. The process according to claim 7, that further comprises previously preparing "in situ" an intermediate of formula (IV), by reaction of an intermediate of formula (VI) 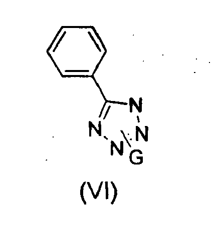 wherein G is as defined above,
with an alkyllithium compound of formula R⁴-Li, wherein R⁴ is a C₁-C₆ linear or branched alkyl, and a boronic ester of formula B(OR⁵)₃, wherein R⁵ is a (C₁-C₄)-alkyl group.

10. The process according to claim 9, wherein G is H.

11. The process according to any of claims 1 to 6, wherein the intermediate (II) or an acid addition salt thereof is prepared by reaction of an intermediate of formula (VII) 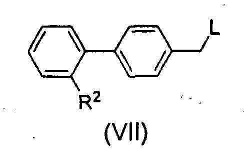 wherein:
R² is a tetrazolyl group or an intermediate or protected form that can be transformed into a tetrazolyl group,
L is a leaving group,
with hexamethylenetetramine in presence of an appropriate solvent system to afford the compound of formula (VIII): 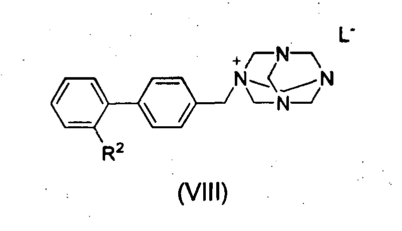 and transforming this compound in acid media to afford compound (II) and optionally transforming said intermediate or protected form of R² into a tetrazolyl group and if desired converting the compound of formula (II) into an acid addition salt thereof.

## Patentansprüche

1. Verfahren zur Herstellung einer Verbindung der Formel (I) oder eines pharmazeutisch verträglichen Salzes davon 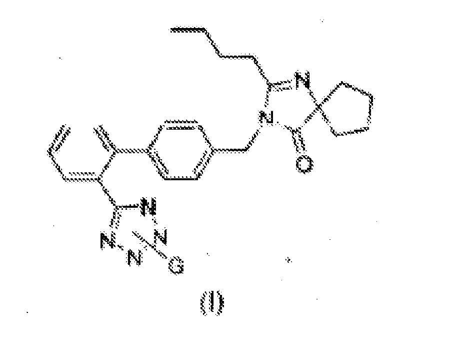 worin:
G Wasserstoff oder eine Tetrazol-Schutzgruppe ist,
bei dem ein Zwischenprodukt der Formel (II) oder ein Säureadditionssalz davon 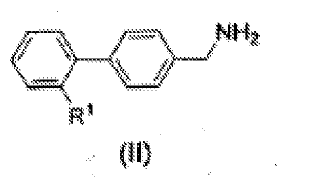 worin:
R¹ eine Tetrazolylgruppe oder ein Zwischenprodukt oder eine geschützte Form, die in eine Tetrazolylgruppe überführt werden kann, ist
mit einem Zwischenprodukt der Formel (III) 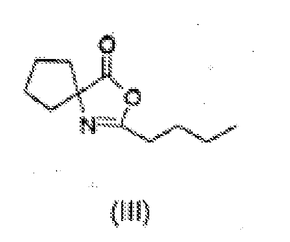 in einem geeigneten Lösungsmittelsystem zur Reaktion gebracht wird, und danach, wenn nötig, die besagten Zwischenprodukte oder geschützten Formen von R¹ in eine Tetrazolylgruppe überführt werden und, nach Wunsch, die besagte Verbindung der Formel (I) nach Wunsch zu einem pharmazeutisch verträglichen Salz derselben umgesetzt wird.

2. Verfahren nach Anspruch 1, bei dem R¹ eine Tetrazolylgruppe und G Wasserstoff ist.

3. Verfahren nach einem der Ansprüche 1 oder 2, bei dem die Reaktion in Gegenwart eines sauren Katalysators ausgeführt wird.

4. Verfahren nach Anspruch 3, bei dem der besagte saure Katalysator aus der Gruppe ausgewählt wird, die aus Methansulfonsäure, *p*-Toluolsulfonsäure und Salzsäure besteht.

5. Verfahren nach einem der Ansprüche 1 bis 4, bei dem das besagte Lösungsmittelsystem ein aprotisch-polares Lösungsmittel enthält.

6. Verfahren nach einem der vorstehenden Ansprüche, bei dem das Zwischenprodukt der Formel (III) hergestellt wird, indem man Cycloleucin und Valeroylchlorid zur Reaktion bringt.

7. Verfahren nach einem der vorstehenden Ansprüche, bei dem das Zwischenprodukt der Formel (II) oder ein Säureadditionssalz davon hergestellt wird, indem man ein Zwischenprodukt der Formel (V) 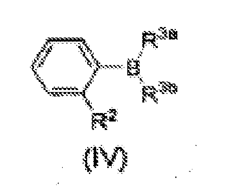 worin:
R² eine Tetrazolylgruppe oder ein Zwischenprodukt oder eine geschützte Form, die in eine Tetrazolylgruppe überführt werden können, ist,
R^{3a} und R^{3b} jeweils unabhängig voneinander aus der Gruppe bestehend aus Cl, Br, (C₁-C₄)-Alkoxyl, Hydroxy, oder als Alternative dazu, ausgewählt werden,
R^{3a} und R^{3b} mit dem B-Atom zusammen eine cyclische Struktur bilden können, die aus einer der folgenden Strukturen ausgewählt wird 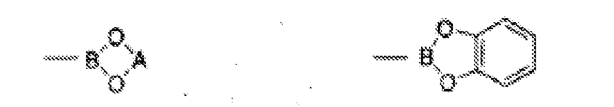
worin A (CH₂)ₙ ist und n eine ganze Zahl von 2 bis 4 ist,
mit einem Zwischenprodukt der Formel (V) oder einem Säureadditionssalz davon 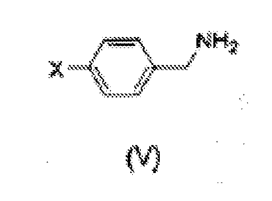 worin:
X eine Abgangsgruppe ist
in Gegenwart einer Base, eines Metallkatalysators und eines geeigneten Lösungsmittelsystems zur Reaktion gebracht wird, und das besagte Zwischenprodukt oder die geschützte Form von R² wahlweise in eine Tetrazolylgruppe überführt wird und nach Wunsch die Verbindung der Formel (II) zu einem Säureadditionssalz derselben umgesetzt wird.

8. Verfahren nach Anspruch 7, bei dem R^{3a} und R^{3b} Hydroxy sind und R² eine Tetrazolylgruppe ist.

9. Verfahren nach Anspruch 7, bei dem weiterhin im Vorfeld ein Zwischenprodukt der Formel (IV) "in situ" hergestellt wird, indem man ein Zwischenprodukt der Formel (VI) 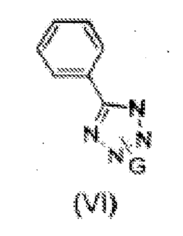 worin G der obenstehenden Definition entspricht, mit einer Alkyllithium-Verbindung der Formel R⁴-Li, worin R⁴ ein unverzweigtes oder verzweigtes (C₁-C₆)-Alkyl ist, und einem Boronsäureester der Formel B(OR⁵)₃, worin R⁵ eine (C₁-C₄)-Alkylgruppe ist, zur Reaktion gebracht wird.

10. Verfahren nach Anspruch 9, wobei G Wasserstoff ist.

11. Verfahren nach einem der Ansprüche 1 bis 6, bei dem das Zwischenprodukt (II) oder ein Säureadditionssalz davon hergestellt wird, indem ein Zwischenprodukt der Formel (VII) 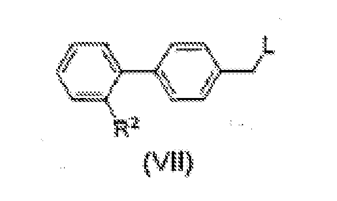 worin:
R² eine Tetrazolylgruppe oder ein Zwischenprodukt oder eine geschützte Form, die in eine Tetrazolylgruppe überführt werden können, ist,
L eine Abgangsgruppe ist,
mit Hexamethylentetramin in Gegenwart eines geeigneten Lösungsmittelsystems zur Reaktion gebracht wird, um die Verbindung der Formel (VIII) hervorzubringen: 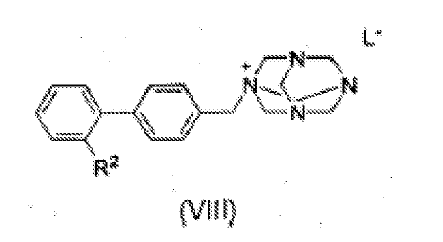 und diese Verbindung in saurer Umgebung umgesetzt wird, um die Verbindung (II) hervorzubringen, und das besagte Zwischenprodukt oder die besagte geschützte Form von R² wahlweise in eine Tetrazolylgruppe überführt wird und die Verbindung der Formel (II) nach Wunsch zu einem Säureadditionssalz davon umgesetzt wird.

## Revendications

1. Procédé pour préparer un composé de formule (I) ou un sel acceptable sur le plan pharmaceutique de celui-ci 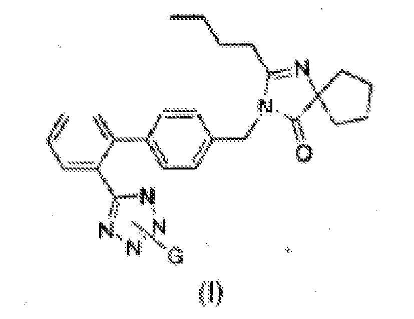 où :
G est H ou un groupe de protection tétrazole,
comprenant la réaction entre un intermédiaire de formule (II) ou un sel d'addition d'acide de celui-ci 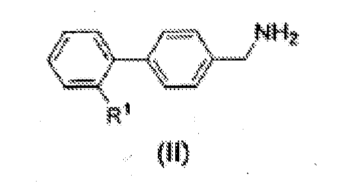 où :
R¹ est un groupe tétrazolyle ou une forme intermédiaire ou protégée qui peut être transformée en un groupe tétrazolyle
et un intermédiaire de formule (III) 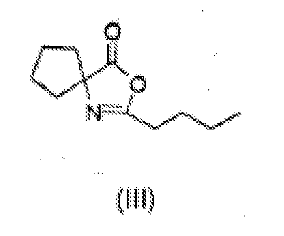 dans un système de solvant approprié et puis selon le besoin la transformation desdites formes intermédiaires ou protégées de R¹ en un groupe tétrazolyle et, si désiré, la conversion dudit composé de formule (I) en un sel acceptable sur le plan pharmaceutique de celui-ci.

2. Procédé selon la revendication 1, dans lequel R¹ est un groupe tétrazolyle et G est H.

3. Procédé selon l'une quelconque des revendications 1 ou 2, dans lequel la réaction est effectuée en présence d'un catalyseur acide.

4. Procédé selon la revendication 3, dans lequel ledit catalyseur acide est choisi dans le groupe constitué de : l'acide méthanesulfonique, l'acide *p*-toluènesulfonique et l'acide chlorhydrique.

5. Procédé selon l'une quelconque des revendications 1 à 4, dans lequel ledit système de solvant comprend un solvant polaire aprotique.

6. Procédé selon l'une quelconque des revendications précédentes, dans lequel l'intermédiaire de formule (III) est préparé par réaction entre la cycloleucine et le chlorure de valéroyle.

7. Procédé selon l'une quelconque des revendications précédentes, dans lequel l'intermédiaire de formule (II) ou un sel d'addition d'acide de celui-ci est préparé par réaction entre un intermédiaire de formule (IV) 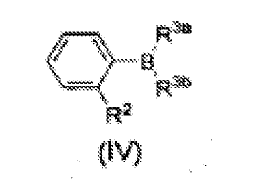 où :
R² est un groupe tétrazolyle ou une forme intermédiaire ou protégée qui peut être transformée en un groupe tétrazolyle,
R^{3a} et R^{3b} sont chacun indépendamment choisis parmi le groupe constitué de : Cl, Br, alcoxy en (C₁ à C₄), hydroxy, ou selon une autre possibilité,
R^{3a} et R^{3b} peuvent être pris conjointement avec l'atome B pour former une structure cyclique choisie parmi une des suivantes 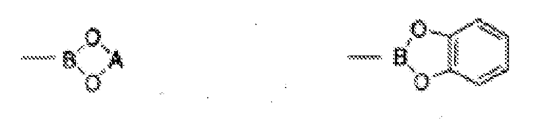
où A est (CH₂)ₙ et n est un nombre entier allant de 2 à 4,
et un intermédiaire de formule (V) ou un sel d'addition d'acide de celui-ci 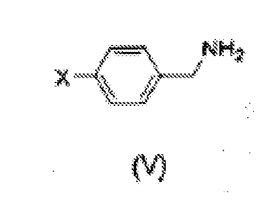 où :
X est un groupe partant
en présence d'une base, un catalyseur métallique et un système de solvant approprié et une transformation éventuelle de ladite forme intermédiaire ou protégée de R² en un groupe tétrazolyle et si désiré la conversion du composé de formule (II) en un sel d'addition d'acide de celui-ci.

8. Procédé selon la revendication 7, dans lequel R^{3a} et R^{3b} sont un hydroxy et R² est un groupe tétrazolyle.

9. Procédé selon la revendication 7, qui comprend en outre la préparation préalable « in situ » d'un intermédiaire de formule (IV), par réaction d'un intermédiaire de formule (VI) 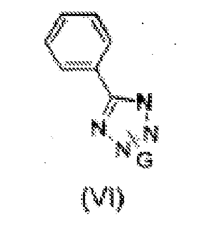 où G est tel que défini précédemment,
avec un composé alkyl-lithium de formule R⁴-Li, dans laquelle R⁴ est un alkyle linéaire ou ramifié en C₁ à C₆, et un ester boronique de formule B(OR⁵)₃, dans laquelle R⁵ est un groupe alkyle en (C₁ à C₄).

10. Procédé selon la revendication 9, dans lequel G est H.

11. Procédé selon l'une quelconque des revendications 1 à 6, dans lequel l'intermédiaire (II) ou le sel d'addition d'acide de celui-ci est préparé par réaction d'un intermédiaire de formule (VII) 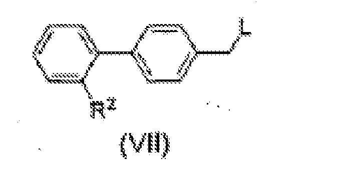 dans laquelle :
R² est un groupe tétrazolyle ou une forme intermédiaire ou protégée qui peut être transformée en un groupe tétrazolyle,
L est un groupe partant,
avec de l'hexaméthylène-tétramine en présence d'un système de solvant approprié pour donner le composé de formule (VIII) : 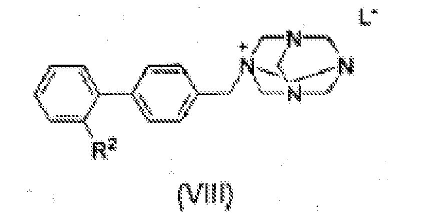 et la transformation de ce composé dans un milieu acide pour donner le composé (II) et éventuellement la transformation de ladite forme intermédiaire ou protégée de R² en un groupe tétrazolyle et si désiré la conversion du composé de formule (II) en un sel d'addition d'acide de celui-ci.
